# EUROPEAN PATENT APPLICATION

(11) **EP 4 810 522 A1**
(43) Date of publication of application: **23.09.2026**
(21) Application number: 24890649.7
(22) Date of filing: 12.11.2024
(51) Int. Cl.: C07K 19/00

(54) **FUSION PROTEIN AND USE THEREOF**

(30) Priority: 14.11.2023 CN 202311516762
(71) Applicant: WeCareLife Biotech Co., Ltd., Beijing 102629 (CN)
(72) Inventor: CAO, Zhen, Beijing 102629 (CN); ZHOU, Chang, Beijing 100101 (CN); ZHANG, Qi, Beijing 100101 (CN); PAN, Bin, Beijing 100101 (CN); ZHI, Zelun, Beijing 100101 (CN)
(74) Representative: Eisenführ Speiser
(86) International application number: PCT/CN2024/131501
(87) International publication number: WO 2025/103294

(57) **Abstract**

The invention relates to the technical field of biology, in particular to a fusion protein and a use thereof. A polypeptide fragment for targeted identification of a monomolecular phospholipid membrane and a polypeptide fragment for targeted identification of a tissue or cell are connected, and the formed fusion protein can specifically bound to the surface of a fat body in a non-covalent mode, such that accurate targeting of the fat body is achieved. The targeted fat body constructed by the fat body targeting peptide fragment can be widely used in fields of precise drug delivery, vaccine preparation etc., and is used for treating diseases such as cancer, infectious diseases and metabolic diseases. Lung tissues and breast cancer cells are used as targets, so that good targeting of the fat body is achieved.

## Description

This application claims the priority of Chinese Patent Application No. 202311516762.2, filed with the China National Intellectual Property Administration on November 14, 2023, and titled with "FUSION PROTEIN AND USE THEREOF", which is hereby incorporated by reference in its entirety.

### FIELD

The present disclosure relates to the field of biotechnology, and in particular to fusion proteins and uses thereof.

### BACKGROUND

An adiposome is a nanoparticle comprising a neutral lipid core surrounded by a monomolecular layer phospholipid membrane or named monolayer phospholipid membrane, which is similar with the structure of naturally occurring lipid droplets and lipoproteins. Adiposomes can recruit one or more resident proteins and/or functional proteins to form artificial lipid droplets, and can also recruit one or more apolipoproteins to form artificial lipoproteins, all of which play an important role in the manufacture of drugs and/or drug carriers. As a novel nanocarrier, the neutral lipid core of an adiposome can encapsulate a large amount of lipophilic drugs and has good biocompatibility.

Similar to the defects of nanocarriers currently used in clinical practice, such as albumin and liposome, adiposomes also lack targeting capability. Therefore, drugs using adiposomes as carriers are delivered to normal tissues of the body, which may lead to many side effects. Therefore, nanocarriers with targeting capability can effectively deliver drugs to diseased tissues or cells, reducing drug damage to healthy tissues, thereby facilitating better treatment of diseases such as cancer, infectious diseases, and metabolic diseases.

Currently, methods commonly used to achieve targeting of nanocarriers are covalent surface modifications, wherein targeting ligands or antibodies are fixed to nanoparticle surfaces through covalent bonds, such as adding glycosyl-modified PEGylated phospholipids and cell-penetrating peptide-modified PEGylated phospholipids to liposomes to target them to brain tissue. However, as a novel nanoparticle, an adiposome is a nanoparticle comprising a neutral lipid core surrounded by a monolayer phospholipid membrane, and existing nanocarrier modification techniques for targeting do not work well on adiposomes. Therefore, there is still an urgent need for methods and strategies specific to adiposome targeting.

### SUMMARY

In view of the above, the technical problem to be solved by the present disclosure is to provide fusion proteins and uses thereof. In the present disclosure, through construction of a fusion protein for adiposome targeting and recruiting them to adiposomes, targeting capability of adiposomes is achieved. The targeting adiposome can be used in fields such as drug delivery and vaccine preparation, thereby being used for the treatment of diseases such as cancers, infectious diseases, and metabolic diseases.

The present disclosure provides a fusion protein comprising fragment A and fragment B, wherein:
fragment A recognizes and targets a monolayer phospholipid membrane;
fragment B recognizes and targets a tissue or a cell.

The fusion protein of the present disclosure comprises at least one fragment A and at least one fragment B, and the present disclosure does not limit the connection order thereof.

In the present disclosure, the structure of the fusion protein is:
[(fragment A)x-(fragment B)y]z or [(fragment B)x-(fragment A)y]z;
wherein: x represents the number of repeats of fragment A, y represents the number of repeats of fragment B, and z represents the number of repeats of a unit composed of fragment A and fragment B in the fusion protein. x, y, and z are each independently an integer not equal to 0. For example, 1≤x≤10, 1≤y≤10, 1≤z≤10. x, y, and z may be equal or may not be equal, and the present disclosure does not limit this. Taking x=1, y=1, and z=1 as an example, the structure of the fusion protein of the present disclosure is fragment A-fragment B or fragment B-fragment A.

When the number of repeats of a fragment is not 1, the sequences of two repeated fragments may be the same or different, and the present disclosure does not limit this. For example, when y=2, the two B fragments may target the same tissue or cell, or may target different tissues or cells, and the present disclosure does not limit this. When z=2, the fragments in the two repeated units may be the same or different, and the present disclosure also does not limit this. Taking x=1, y=2, and z=2 as an example, the structure of the fusion protein of the present disclosure is:
[fragment A-fragment B₂]₂, i.e.: fragment A - fragment B - fragment B - fragment A - fragment B - fragment B;
or [fragment B-fragment A₂]₂, i.e.: fragment B-fragment A-fragment A-fragment B-fragment A-fragment A.

In order to maintain the structural integrity of fragment A and fragment B, the fusion protein may or may not comprise a linker, and the present disclosure does not limit this. In the present disclosure, the linker is located between two adjacent fragments, for example, between fragment A and fragment B, between two A fragments, or between two B fragments, and the present disclosure does not limit this. Optionally, the length of the linker is 2 to 20 amino acids, or 5 to 15 amino acids, or 10 to 15 amino acids, and the present disclosure also does not limit this. For example, the linker may be (GGGGS)n, Gn, (EAAAK)n, (XP)n, A(EAAAK)₄ALEA(EAAAK)₄A, PAPAP, VSQTSKLTRAETVFPDV, PLGLWA, TRHRQPRGWE, AGNRVRRSVG, RVLAEA, EDVVCCSMSY, EDVVCCSMSY, or GGIEGRGS, wherein n is an integer not equal to zero. Alternatively, the linker is a cleavable linker or a self-cleaving linker. The amino acid sequence of the cleavable linker may be LEAGCKNFFPRSFTSCGSLE, and the self-cleaving linker may be P2A, T2A, or E2A.

In the present disclosure, fragment A can target a monolayer phospholipid membrane. Since adiposomes have the significant characteristic of having a monolayer phospholipid membrane, fragment A is capable of targeting adiposomes.

In some embodiments, the monolayer phospholipid membrane targeted by fragment A comprises one or more selected from the group consisting of phospholipids, functional polar lipids, and cationic lipids;
the phospholipid is one or more selected from the group consisting of phosphatidylcholine, phosphatidylethanolamine, phosphatidylinositol, phosphatidylserine, phosphatidylglycerol, phosphatidic acid, cardiolipin, and sphingomyelin;
the functional polar lipid is one or more selected from the group consisting of PEGylated sterol, biotinylated sterol, amino acid-modified sterol, peptide-modified sterol, polysaccharide-modified sterol, nucleic acid-modified sterol, PEGylated phospholipid, biotinylated phospholipid, amino acid-modified phospholipid, peptide-modified phospholipid, polysaccharide-modified phospholipid, and nucleic acid-modified phospholipid;
and the cationic lipid is one or more selected from the group consisting of (2,3-dioleoyl-propyl)-trimethylammonium-chloride, (2,3-dioleoyl-propyl)- trimethylammonium, 2,3-dioleoyloxy-N-[2-(sperminecarbamoylamino)ethyl]-N,N-dimethyl-1-propanamine hydrochloride, 1,2-dioleoyl-sn-glycero-3-[(N-(5-amino-1-carboxypentyl)iminodiacetic acid)succinyl](nickel salt), and 3β-[N-(N',N'-dimethylaminoethane)-carbamoyl]cholesterol hydrochloride.

The peptides targeting the monolayer phospholipid membrane are usually a fragment of a lipid droplet protein, an artificially designed peptide capable of localizing to cellular lipid droplets, or a fragment of an apolipoprotein. Apolipoproteins and peptides capable of localizing to lipid droplets can be used as peptides that specifically recognize a monolayer phospholipid membrane (fragment A). The following are peptides capable of specifically binding to an adiposome surface in a non-covalent manner, including but not limited to AAMB, ALDI, CYB5R3-N, LDAMP1, HSD17B13-N28, MDT-28-P, MLDS-P, DHS-3-P, HSD17B11-N28, PspA-H1, Vipp1-H1, Snf7-H1, Chmp1B-H1, PB, PE, and PF.

**Table 1 Names and amino acid sequences of fragment A**

| Fragment A | Amino acid sequence |
|---|---|
| AAMB | MELTIFILRLAIYILTFPLYLLNFLGLW (SEQ ID NO:1) |
| ALDI | MDALVLFLQLLVLLLTLPLHLLALLGC (SEQ ID NO:2) |
| CYB5R3-N | MGAQLSTLGHMVLFPVWFLYSLLMKLFQ (SEQ ID NO:3) |
| LDAMP1 | MQIPVLTRILPAQLLLWSRVRLWRLMMWLWPLQKILLQLVSH (SEQ ID NO:4) |
| HSD17B13-N28 | MNIILEILLLLITIIYSYLESLVKFFIP (SEQ ID NO:5) |
| MDT-28-P | MLTIGQRVKNLASLIVQGVSNKAHDHVIDPINERARNYLE (SEQ ID NO:6) |
| MLDS-P | MPEELAELREKFTADELRKVAEAYLKVASDLYTSLAERGEDT VERIRKQPVVEEGIGRAETAFGD (SEQ ID NO:7) |
| DHS-3-P | MQDILESVVVTLHAICLLIISTIRNCFPMGWLLRKDVRGQTVL ITGSGSGL (SEQ ID NO:8) |
| HSD17B11-N28 | MKFLLDILLLLPLLIVCSLESFVKLFIPKRRKSVTGEI (SEQ ID NO:9) |
| PspA-H1 | MGIFSRFADIVNANINALLEK (SEQ ID NO:10) |
| Vipp1-H1 | MNLFERFSRVVKSYANALISSF (SEQ ID NO:11) |
| Snf7-H1 | MWSSLFGWTSSNAKNKESPTKAIVRLREHINLLSKKQSHLRT QITNQEN (SEQ ID NO:12) |
| Chmp1B-H1 | MAVNFLRMSARVDAVAARVQTAVTMGKVTK (SEQ ID NO:13) |
| PB | MRRLLRILRLLRRIIRRRLR (SEQ ID NO:14) |
| PE | MRFLRRILRLLRRIIRRVLR (SEQ ID NO: 15) |
| PF | MEFLESVLSGLVRIIRRVLE (SEQ ID NO: 16) |

The amino acid sequences of some fragment A of the present disclosure are as shown in any item of Table 1, or has at least 80% (preferably 85%, 90%, 95%, 98%, or 99%) sequence identity to the amino acid sequence of the polypeptide described in Table 1.

In the present disclosure, fragment B recognizes and targets tissues or cells. The tissues or cells are derived from human or animal bodies and may be tumors, pathogen-infected organs, lesions of metabolic diseases, and/or healthy tissues or cells. In the present disclosure, the selection of peptides targeting specific tissues or cells depends on the specific proteins on the surface of the tissues or cells to be targeted. In some embodiments, the fragment B includes, but is not limited to: at least one of Nrp-B, Trf-B, LDLR-B, ErbB2-B, CXCR4-B, GRP78-B, and Soma-B.

**Table 2 Names and amino acid sequences of fragment B and targeting proteins**

| Fragment B Name | Amino acid sequence | Targeting protein |
|---|---|---|
| Nrp-B | CRGDK (SEQ ID NO:17) | Neuropilin-1 |
| Trf-B | THRPPMWSPVWP (SEQ ID NO:18) | Transferrin receptor 1 |
| LDLR-B | HLRKLRKRLLR (SEQ ID NO:19) | Low-density lipoprotein receptor |
| ErbB2-B | FCDGFYACYKDV (SEQ ID NO:20) | Erythroblastic leukemia viral oncogene homolog 2 |
| CXCR4-B | LGASWHRPDK (SEQ ID NO:21) | C-X-C chemokine receptor type 4 |
| GRP78-B | CTVALPGGYVRVC (SEQ ID NO:22) | Glucose-regulated protein 78 |
| Soma-B | FCDWKTCT (SEQ ID NO:23) | Somatostatin |

The amino acid sequences of some fragment B of the present disclosure are as shown in any item of Table 2, or is a sequence having at least 80% (preferably 85%, 90%, 95%, 98%, or 99%) sequence identity to the amino acid sequence of the polypeptide described in Table 2.

Optionally, the fusion protein of the present disclosure is formed by connecting fragment A and fragment B, for example, the fusion protein is:
AAMB-Nrp-B, AAMB-Trf-B, AAMB-LDLR-B, AAMB-ErbB2-B, AAMB-CXCR4-B, AAMB-GRP78-B, AAMB-Soma-B, Nrp-B-AAMB, Trf-B-AAMB, LDLR-B-AAMB, ErbB2-B-AAMB, CXCR4-B-AAMB, GRP78-B-AAMB, Soma-B-AAMB, ALDI-Nrp-B, ALDI-Trf-B, ALDI-LDLR-B, ALDI-ErbB2-B, ALDI-CXCR4-B, ALDI-GRP78-B, ALDI-Soma-B, Nrp-B-ALDI, Trf-B-ALDI, LDLR-B-ALDI, ErbB2-B-ALDI, CXCR4-B-ALDI, GRP78-B-ALDI, Soma-B-ALDI, CYB5R3-N-Nrp-B, CYB5R3-N-Trf-B, CYB5R3-N-LDLR-B, CYB5R3-N-ErbB2-B, CYB5R3-N-CXCR4-B, CYB5R3-N-GRP78-B, CYB5R3-N-Soma-B, Nrp-B-CYB5R3-N, Trf-B-CYB5R3-N, LDLR-B-CYB5R3-N, ErbB2-B-CYB5R3-N, CXCR4-B-CYB5R3-N, GRP78-B-CYB5R3-N, Soma-B-CYB5R3-N, LDAMP1-Nrp-B, LDAMP1-Trf-B, LDAMP1-LDLR-B, LDAMP1-ErbB2-B, LDAMP1-CXCR4-B, LDAMP1-GRP78-B, LDAMP1-Soma-B, Nrp-B-LDAMP1, Trf-B-LDAMP1, LDLR-B-LDAMP1, ErbB2-B-LDAMP1, CXCR4-B-LDAMP1, GRP78-B-LDAMP1, Soma-B-LDAMP1, HSD17B13-N28-Nrp-B, HSD17B13-N28-Trf-B, HSD17B13-N28-LDLR-B, HSD17B13-N28-ErbB2-B, HSD17B13-N28-CXCR4-B, HSD17B13-N28-GRP78-B, HSD17B13-N28-Soma-B, Nrp-B-HSD17B13-N28, Trf-B-HSD17B13-N28, LDLR-B-HSD17B13-N28, ErbB2-B-HSD17B13-N28, CXCR4-B-HSD17B13-N28, GRP78-B-HSD17B13-N28, Soma-B-HSD17B13-N28, MDT-28-P-Nrp-B, MDT-28-P-Trf-B, MDT-28-P-LDLR-B, MDT-28-P-ErbB2-B, MDT-28-P-CXCR4-B, MDT-28-P-GRP78-B, MDT-28-P-Soma-B, Nrp-B-MDT-28-P, Trf-B-MDT-28-P, LDLR-B-MDT-28-P, ErbB2-B-MDT-28-P, CXCR4-B-MDT-28-P, GRP78-B-MDT-28-P, Soma-B-MDT-28-P, MLDS-P-Nrp-B, MLDS-P-Trf-B, MLDS-P-LDLR-B, MLDS-P-ErbB2-B, MLDS-P-CXCR4-B, MLDS-P-GRP78-B, MLDS-P-Soma-B, Nrp-B-MLDS-P, Trf-B-MLDS-P, LDLR-B-MLDS-P, ErbB2-B-MLDS-P, CXCR4-B-MLDS-P, GRP78-B-MLDS-P, Soma-B-MLDS-P, DHS-3-P-Nrp-B, DHS-3-P-Trf-B, DHS-3-P-LDLR-B, DHS-3-P-ErbB2-B, DHS-3-P-CXCR4-B, DHS-3-P-GRP78-B, DHS-3-P-Soma-B, Nrp-B-DHS-3-P, Trf-B-DHS-3-P, LDLR-B-DHS-3-P, ErbB2-B-DHS-3-P, CXCR4-B-DHS-3-P, GRP78-B-DHS-3-P, Soma-B-DHS-3-P, HSD17B11-N28-Nrp-B, HSD17B11-N28-Trf-B, HSD17B11-N28-LDLR-B, HSD17B11-N28-ErbB2-B, HSD17B11-N28-CXCR4-B, HSD17B11-N28-GRP78-B, HSD17B11-N28-Soma-B, Nrp-B-HSD17B11-N28, Trf-B-HSD17B11-N28, LDLR-B-HSD17B11-N28, ErbB2-B-HSD17B11-N28, CXCR4-B-HSD17B11-N28, GRP78-B-HSD17B11-N28, Soma-B-HSD17B11-N28, PSPA-H1-Nrp-B, PSPA-H1-Trf-B, PSPA-H1-LDLR-B, PSPA-H1-ErbB2-B, PSPA-H1-CXCR4-B, PSPA-H1-GRP78-B, PSPA-H1-Soma-B, Nrp-B-PSPA-H1, Trf-B-PSPA-H1, LDLR-B-PSPA-H1, ErbB2-B-PSPA-H1, CXCR4-B-PSPA-H1, GRP78-B-PSPA-H1, Soma-B-PSPA-H1, VIPP1-H1-Nrp-B, VIPP1-H1-Trf-B, VIPP1-H1-LDLR-B, VIPP1-H1-ErbB2-B, VIPP1-H1-CXCR4-B, VIPP1-H1-GRP78-B, VIPP1-H1-Soma-B, Nrp-B-VIPP1-H1, Trf-B-VIPP1-H1, LDLR-B-VIPP1-H1, ErbB2-B-VIPP1-H1, CXCR4-B-VIPP1-H1, GRP78-B-VIPP1-H1, Soma-B-VIPP1-H1, SNF7-H1-Nrp-B, SNF7-H1-Trf-B, SNF7-H1-LDLR-B, SNF7-H1-ErbB2-B, SNF7-H1-CXCR4-B, SNF7-H1-GRP78-B, SNF7-H1-Soma-B, Nrp-B-SNF7-H1, Trf-B-SNF7-H1, LDLR-B-SNF7-H1, ErbB2-B-SNF7-H1, CXCR4-B-SNF7-H1, GRP78-B-SNF7-H1, Soma-B-SNF7-H1, CHMP1B-H1-Nrp-B, CHMP1B-H1-Trf-B, CHMP1B-H1-LDLR-B, CHMP1B-H1-ErbB2-B, CHMP1B-H1-CXCR4-B, CHMP1B-H1-GRP78-B, CHMP1B-H1-Soma-B, Nrp-B-CHMP1B-H1, Trf-B-CHMP1B-H1, LDLR-B-CHMP1B-H1, ErbB2-B-CHMP1B-H1, CXCR4-B-CHMP1B-H1, GRP78-B-CHMP1B-H1, Soma-B-CHMP1B-H1, PB-Nrp-B, PB-Trf-B, PB-LDLR-B, PB-ErbB2-B, PB-CXCR4-B, PB-GRP78-B, PB-Soma-B, Nrp-B-PB, Trf-B-PB, LDLR-B-PB, ErbB2-B-PB, CXCR4-B-PB, GRP78-B-PB, Soma-B-PB, PE-Nrp-B, PE-Trf-B, PE-LDLR-B, PE-ErbB2-B, PE-CXCR4-B, PE-GRP78-B, PE-Soma-B, Nrp-B-PE, Trf-B-PE, LDLR-B-PE, ErbB2-B-PE, CXCR4-B-PE, GRP78-B-PE, Soma-B-PE, PF-Nrp-B, PF-Trf-B, PF-LDLR-B, PF-ErbB2-B, PF-CXCR4-B, PF-GRP78-B, PF-Soma-B, Nrp-B-PF, Trf-B-PF, LDLR-B-PF, ErbB2-B-PF, CXCR4-B-PF, GRP78-B-PF, or Soma-B-PF.

Further, the present disclosure also provides biological materials related to the fusion protein, comprising any one of the following:
I) a nucleic acid encoding the fusion protein;
II) an expression unit containing the nucleic acid described in I);
III) a vector containing the nucleic acid described in I) or the expression unit described in II);
IV) a host cell transformed or transfected with the vector described in III);
V) an expression product of the host cell described in IV).

The present disclosure also provides a method for producing the fusion protein. The fusion protein can be produced by genetic engineering methods or chemical synthesis methods.

In some embodiments, the production method comprises:
culturing the host cell to obtain the fusion protein;
or coupling fragment A and fragment B by biological and/or chemical methods;
or synthesizing the fusion protein by chemical synthesis.

The coupling optionally comprises the use of a linker.

The chemical synthesis may be used in the present disclosure includes solid-phase synthesis, liquid-phase synthesis, and solid-phase-liquid-phase combined synthesis, and the present disclosure is not limited thereto.

The fusion protein of the present disclosure can specifically bind to the adiposome surface in a non-covalent manner, thereby achieving specific targeting of adiposomes. Targeting adiposomes constructed by the targeting peptides can be widely used in fields such as precise drug delivery and vaccine manufacture, thereby being used for the treatment of diseases such as cancers, infectious diseases, and metabolic diseases. Based on this, the present disclosure further provides a use of the fusion proteins in the manufacture of targeting adiposomes.

Further, the present disclosure also provides a targeting adiposome, wherein the preparation raw materials comprise: the aforementioned fusion proteins, polar lipids, and neutral lipids.

Optionally, the polar lipids include one or more of phospholipids, functional polar lipids, and cationic lipids; wherein
the phospholipid is one or more selected from the group consisting of phosphatidylcholine, phosphatidylethanolamine, phosphatidylinositol, phosphatidylserine, phosphatidylglycerol, phosphatidic acid, cardiolipin, and sphingomyelin;
the functional polar lipid is one or more selected from the group consisting of PEGylated sterol, biotinylated sterol, amino acid-modified sterol, peptide-modified sterol, polysaccharide-modified sterol, nucleic acid-modified sterol, PEGylated phospholipid, biotinylated phospholipid, amino acid-modified phospholipid, peptide-modified phospholipid, polysaccharide-modified phospholipid, and nucleic acid-modified phospholipid;
the cationic lipid is one or more selected from the group consisting of (2,3-dioleoyl-propyl)-trimethylammonium-chloride, (2,3-dioleoyl-propyl)- trimethylammonium, 2,3-dioleoyloxy-N-[2-(sperminecarboxamido)ethyl]-N,N-dimethyl-1-propanamine hydrochloride, 1,2-dioleoyl-sn-glycero-3-[(N-(5-amino-1-carboxypentyl)iminodiacetic acid)succinyl](nickel salt), and 3β-[N-(N',N'-dimethylaminoethane)-carbamoyl]cholesterol hydrochloride;
the neutral lipid is one or more selected from the group consisting of triglyceride, wax ester, sterol ester, steryl ester, retinol ester, and fat-soluble vitamin.

Optionally, the raw materials further comprise a solvent, wherein the solvent comprises an organic solvent and a buffer. The organic solvent includes, but is not limited to, methanol, ethanol, isopropanol, diethyl ether, chloroform, ethyl acetate, dichloromethane, trichloromethane, benzene, toluene, xylene, methane, acetonitrile, pyridine, or phenol. The buffer includes, but is not limited to, citrate buffer, phosphate buffer, and acetate buffer.

The targeting adiposome of the present disclosure can deliver a drug having a therapeutic function to a target site. Optionally, the raw materials further comprise a drug. In the present disclosure, the drug is a substance for preventing, treating, and diagnosing diseases, comprising but not limited to drugs for preventing and treating the following diseases: cancer, infectious disease, cardiovascular disease, and metabolic diseases such as diabetes.

In addition, the targeting adiposome can further comprise a marker having an indicator function, such as a fluorescent protein.

Further, the present disclosure also provides a method for producing the targeting adiposome, comprising:
dissolving thefusion protein to prepare a solution A;
dissolving a polar lipid to prepare a solution B;
mixing the solution A and the solution B, followed by drying, redissolving, then mixing with a neutral lipid, and centrifuging followed by centrifugal separation to obtain a product containing the targeting adiposomes.

In some embodiments of the present disclosure, the molar ratio of the polar lipid to the fusion protein is (100 to 100,000):(1 to 10). In some embodiments, the molar ratio of the polar lipid to the fusion protein is (100 to 10,000):(1 to 10), or (100 to 1,000):(1 to 10), or (1,000 to 100,000):(1 to 10), or (10,000 to 100,000):(1 to 10), or 100:(1 to 10), or 1,000:(1 to 10), or 10,000:(1 to 10), or 100,000:(1 to 10), or 100:1, or 1,000:1, or 10,000:1, or 100,000:1, or 100:2, or 1,000:2, or 10,000:2, or 100,000:2, or 100:3, or 1,000:3, or 10,000:3, or 100,000:3, or 100:4, or 1,000:4, or 10,000:4, or 100,000:4, or 100:5, or 1,000:5, or 10,000:5, or 100,000:5, or 100:6, or 1,000:6, or 10,000:6, or 100,000:6, or 100:7, or 1,000:7, or 10,000:7, or 100,000:7, or 100:8, or 1,000:8, or 10,000:8, or 100,000:8, or 100:9, or 1,000:9, or 10,000:9, or 100,000:9, or 100:10, or 1,000:10, or 10,000:10, or 100,000:10.

The solvent of solution A is a mixed solution of methanol and chloroform, wherein the volume ratio of methanol to chloroform is (1 to 10):(1 to 10). For example, the volume ratio is 1:1, 1:2, 1:3, 1:4, 1:5, 1:6, 1:7, 1:8, 1:9, or 1:10. The fusion protein in solution A has a concentration of 0.1 to 10 mg/mL. For example, the fusion protein has a concentration of 0.1 mg/ml, 0.2 mg/ml, 0.3 mg/ml, 0.4 mg/ml, 0.5 mg/ml, 1 mg/ml, 2 mg/ml, 3 mg/ml, 4 mg/ml, 5 mg/ml, 6 mg/ml, 7 mg/ml, 8 mg/ml, 9 mg/ml, or 10 mg/ml.

The solvent of solution B is a 2-di-(9Z-octadecenoyl)-sn-glycero-3-phosphocholine solution. Each 80 µL of solution B contains 2 mg of phospholipid. The volume ratio of solution A to solution B for mixing may be 1:4.

The redissolving step and/or the centrifugal separation step may be performed with reference to Chinese Patent Application No. 201510974956.6.

The method for producing the targeting adiposome of the present disclosure has simple and concise steps, does not require complex instruments, and has strong operability.

Further, the present disclosure also provides the use of the targeting adiposome as described above or the targeting adiposome obtained by the method as described above in the manufacture of a drug or vaccine for preventing and treating diseases.

In some embodiments, the disease is a cancer, a pathogen-induced infectious disease, or a metabolic disease. The disease is preferably a cancer, wherein the cancer may be breast cancer, lung cancer, kidney cancer, laryngeal cancer, liver cancer, muscle tissue cancer, blood cancer, bone cancer, brain cancer, neck cancer, oral or nasal mucosal cancer, bladder cancer, central nervous system cancer, cervical cancer, colon cancer, endometrial cancer, external genitalia cancer, esophageal cancer, gallbladder cancer, gastrointestinal cancer, genitourinary cancer, head cancer, ovarian cancer, pancreatic cancer, prostate cancer, skin cancer, spleen cancer, small intestine cancer, large intestine cancer, stomach cancer, testicular cancer, and/or thyroid cancer.

Further, the present disclosure also provides a drug or vaccine for preventing and treating a disease, comprising a pharmaceutically acceptable excipient and the targeting adiposome as described above or the targeting adiposome produced by the method as described above. The dosage form of the drug or vaccine of the present disclosure may be an oral preparation, an inhalant, or an injection.

Optionally, the drug or vaccine is an oral preparation, for example, a tablet, a pill, an oral liquid, a capsule, a syrup, a dropping pill, or a granule.

In some embodiments provided by the present disclosure, the capsule is a hard capsule or a soft capsule.

In some embodiments provided by the present disclosure, the tablet is an oral tablet or a buccal tablet.

An oral tablet refers to a tablet for oral administration. In most such tablets, the drug exerts its effect after being absorbed through the gastrointestinal tract, and some tablets exerts its effect locally in the gastrointestinal tract. In some embodiments provided by the present disclosure, the oral tablet is a conventional compressed tablet, a dispersible tablet, an effervescent tablet, a chewable tablet, a coated tablet, or a sustained/controlled release tablet.

The drug or vaccine may be an inhalant. Optionally, it is an inhalation aerosol, an inhalation powder, or a liquid preparation for use in a nebulizer.

The drug or vaccine may be an injection. For example, it is an injection solution or a powder for injection.

The present disclosure also provides a method for preventing and treating a disease, comprising administering the drug or vaccine as described above to a subject in need thereof. The administration method comprises oral administration, inhalation, and/or injection.

The present disclosure provides a fusion protein by connecting a polypeptide fragment that recognizes and targets the monolayer phospholipid membrane with a polypeptide fragment that recognizes and targets tissues or cells, which can specifically bind to the adiposome surface in a non-covalent manner, thereby achieving precise targeting of the adiposome. The targeting adiposome constructed by the adiposome targeting peptide can be widely used in fields such as precise drug delivery and vaccine manufacture, thereby being used for the prevention, treatment, and diagnosis of diseases such as cancers, infectious diseases, and metabolic diseases. In some embodiments, as an example, the adiposomes of the present disclosure exhibit good targeting to lung tissue and breast cancer cells.

### BRIEF DESCRIPTION OF DRAWINGS

FIG. 1 shows a schematic diagram of the structure of an adiposome targeting peptide .
FIG. 2 shows the preparation of adiposome targeting peptide LTA-P33.
FIG. 3 shows the construction of adiposomes containing adiposome targeting peptide LTA-P33, wherein:
FIG. 3A shows the results of SDS-PAGE protein gel electrophoresis of adiposomes containing adiposome targeting peptide LTA-P33, wherein the arrow in the LTA adiposome lane indicates the adiposome targeting peptide LTA-P33 adsorbed to the adiposomes;

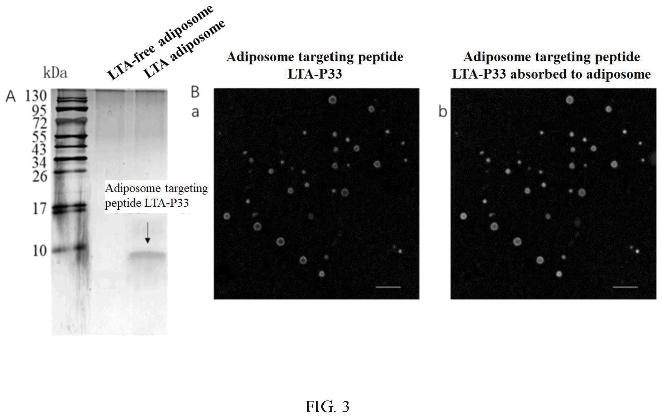

FIG. 3B shows the results of confocal laser scanning microscopy of adiposomes containing adiposome targeting peptide LTA-P33; panel a shows the bright ring-like structure as adiposome targeting peptide LTA-P33; panel b shows adiposomes stained red by neutral lipid-specific dye LipidTox Red (shown as light gray in the figure), with the bright ring-like structure on the surface being adiposome targeting peptide LTA-P33; the scale bar is 5 µm; silver staining and confocal laser scanning microscopy results indicate successful construction of adiposomes containing adiposome targeting peptide LTA-P33, i.e., lung-targeting adiposomes.
FIG. 4 shows that adiposomes containing adiposome targeting peptide LTA-P33 are capable of targeting the lungs, wherein:
FIG. 4A shows the expression levels of Neuropilin-1 protein in heart, lung, spleen, kidney, and liver tissues.
FIG. 4B shows fluorescently labeled LTA-free adiposomes (adiposomes without adiposome targeting peptide LTA-P33) and LTA adiposomes (adiposomes with adiposome targeting peptide LTA-P33) being injected into mice via tail vein, respectively; three hours after injection, the mice were dissected and various tissues were tested; panel a shows the distribution of LTA-free adiposomes in various tissues; panel b shows the distribution of LTA adiposomes in tissues;
FIG. 4C shows the fluorescence statistics of LTA-free adiposomes and LTA adiposomes in lung tissues compared with a blank group;
FIG. 4D shows the distribution of LTA-free adiposomes and LTA adiposomes in various tissues 24 hours (a) and 54 hours (b) after injection.
FIG. 5 shows the preparation of adiposome targeting peptide TRTA-P33.
FIG. 6 shows the construction of adiposomes containing adiposome targeting peptide TRTA-P33; wherein:
FIG. 6A shows the results of SDS-PAGE protein gel electrophoresis of adiposomes containing adiposome targeting peptide TRTA-P33, wherein the arrow in the TRTA adiposome lane indicates the adiposome targeting peptide TRTA-P33 adsorbed to the adiposomes.
FIG. 6B shows the results of confocal laser scanning microscopy of adiposomes containing adiposome targeting peptide TRTA-P33; panel a shows the bright ring-like structure indicating adiposome targeting peptide TRTA-P33; panel b shows adiposomes stained red by neutral lipid-specific dye LipidTox Red (shown as light gray in the figure), with the bright ring-like structure on the surface being adiposome targeting peptide TRTA-P33; the scale bar is 5 µm. Silver staining and confocal laser scanning microscopy results indicate successful construction of adiposomes containing adiposome targeting peptide TRTA-P33, i.e., a transferrin receptor-targeting adiposome.
FIG. 7 shows that adiposomes containing adiposome targeting peptide TRTA-P33 target MCF7 breast cancer cells with high expression of transferrin receptor; wherein:
FIG. 7A shows expression levels of Transferrin receptor 1 protein in different cancer cells.
FIG. 7B shows confocal laser scanning microscopy results after adiposomes without adiposome targeting peptide TRTA-P33 (non-TRTA adiposomes, a) and adiposomes with adiposome targeting peptide TRTA-P33 (TRTA adiposomes, b) were incubated with MCF7 cells, respectively; bright spots are adiposomes containing fluorescent labels; the scale bar is 5 µm; panel a shows the signal intensity of fluorescence-labeled adiposomes in MCF7 cells; panel b shows the statistical results of signal intensity of fluorescence-labeled adiposomes in MCF7 cells;
FIG. 7C shows flow cytometry statistics results after non-TRTA adiposomes (a) and TRTA adiposomes (b) were incubated with MCF7 cells, respectively.

### DETAILED DESCRIPTION

The present disclosure provides fusion proteins and uses thereof. Those skilled in the art can draw on the content herein and appropriately improve process parameters to implement the same. In particular, it should be noted that all similar substitutions and modifications are obvious to those skilled in the art and are all considered to be included in the present disclosure. The methods and applications of the present disclosure have been described through preferred embodiments, and relevant personnel can obviously make modifications or appropriate changes and combinations to the methods and applications herein without departing from the content, spirit, and scope of the present disclosure, thereby implementing and applying the technology of the present disclosure.

Unless otherwise defined, all scientific and technical terms used herein have the same meaning as commonly understood by those of ordinary skill in the art. Regarding definitions and terminology in the art, professionals may specifically refer to Current Protocols in Molecular Biology (Ausubel). The abbreviations for amino acid residues are the standard 3-letter and/or 1-letter codes used in the art to refer to one of the 20 common L-amino acids.

In this application, the term "and/or" describes an associative relationship between associated objects, indicating that three relationships may exist. For example, A and/or B may represent: A exists alone, both A and B exist, or B exists alone. A and B may be singular or plural.

The terms "comprising", "including" and "having" are used interchangeably herein and are intended to be inclusive, meaning that the described solution may include other elements in addition to those listed. It should also be understood that the use of "comprising", "including" and "having" herein also provides solutions "consisting of" the listed elements.

In the present disclosure, "at least one" refers to one or more, and "a plurality of" refers to two or more. "At least one of the following" or similar expressions refer to any combination of these items, including any combination of a single item or a plurality of items.

The term "nucleic acid" herein refers to a nucleic acid encoding the fusion protein as described above, which may be single-stranded or double-stranded, and may be DNA, RNA, cDNA, or PNA. The nucleic acid may include nucleotide sequences having different functions, for example coding regions and non-coding regions such as regulatory sequences (e.g., promoters or transcription terminators). The nucleic acid may be linear or circular in topology. The nucleic acid may be, for example, part of a vector (such as an expression vector or cloning vector), or a combination of one fragment or a plurality of fragments. The nucleic acid may be obtained directly from natural sources, or may be prepared by recombinant, enzymatic, or chemical techniques. The RNA form includes mRNA obtained by transcription of a gene, and the like.

The term "expression unit" herein includes the nucleic acid described in the present disclosure, a promoter and a terminator. To regulate the level of expression or protein folding, the expression unit may further include other elements.

The term "vector" herein refers to a nucleic acid molecule capable of amplifying another nucleic acid linked thereto. This term includes vectors as self-replicating nucleic acid structures and vectors integrated into the genome of a host cell. Certain vectors are capable of directing the expression of nucleic acids linked thereto. Such vectors are referred to herein as "expression vectors". In the present disclosure, the described vectors are used for amplification or preservation of nucleic acids, or for expression of mutants in a host cell. The vectors described in the present disclosure include a backbone and a nucleic acid. Specifically, the backbone is derived from a plant, animal, bacterium, fungus, phage, or virus, which is not limited in the present disclosure.

The term "host cell" herein refers to a cell into which an exogenous nucleic acid has been introduced, including progeny of such a cell. Host cells include "transformants" and "transformed cells", which include the primary transformed cell and progeny derived therefrom, regardless of the number of passages. The progeny may not be completely identical to the parent cell in nucleic acid content but may contain mutations. The present disclosure includes mutant progeny having the same function or biological activity as was screened or selected in the initially transformed cell.

The term "prevention" and "treatment" herein includes prevention and/or treatment. "Treatment" refers to surgical or therapeutic treatment, the purpose of which is to prevent, slow(reduce) the undesirable physiological changes or lesions, such as cancer and tumors, in a subject being treated. Beneficial or desired clinical outcomes include, but are not limited to, alleviation of symptoms, reduction in disease severity, stabilization of disease state (i.e., no worsening), delayed or slowed disease progression, amelioration or palliation of disease state, and alleviation (whether partial or complete), whether detectable or undetectable. Subjects in need of treatment include subjects already suffering from a condition or disease, as well as subjects prone to developing a condition or disease or subjects intending to prevent a condition or disease. When terms such as slowing, reducing, weakening, palliating, or alleviating are mentioned, their meanings also include elimination, disappearance, or non-occurrence.

The term "administering" herein refers to an organism receiving treatment for a specific disease or condition as described in the present disclosure. By way of example, a subject receiving treatment for a disease or symptom may be a human, a primate (e.g., a monkey), or a non-primate mammal.

It should be understood that in various embodiments of the present disclosure, the sequence numbers of the above processes does not imply the order of execution, and some or all steps may be performed in parallel or sequentially. The execution order of each process should be determined by its function and internal logic, and should not constitute any limitation on the implementation process of the embodiments of the present disclosure.

All reagents and materials used in the present disclosure are all commercially available products and can be purchased from the market.

In the examples of the present disclosure, the following two combinations of adiposome targeting peptides were verified. 1) Fragment A is peptide AAMB in Table 1 that specifically recognizes a monolayer phospholipid membrane, and fragment B is peptide Nrp-B in Table 2 that targets Neuropilin-1, a protein highly expressed in lung tissue. The adiposome targeting peptide LTA-P33 (lung-targeting adiposome peptide 33) was constructed by chemical synthesis with a sequence of MELTIFILRLAIYILTFPLYLLNFLGLWCRGDK. Adiposomes constructed using peptide LTA-P33 could specifically target lung tissue. 2) Fragment A is peptide PspA-H1 in Table 1 that specifically recognizes a monolayer phospholipid membrane, and fragment B is peptide Trf-B in Table 2 that targets Transferrin receptor 1, a protein highly expressed in MCF7 breast cancer cells. The adiposome targeting peptide TRTA-P33 (Transferrin receptor 1-targeting adiposome peptide 33) was constructed by chemical synthesis with a sequence of MGIFSRFADIVNANINALLEKTHRPPMWSPVWP. Adiposomes constructed using peptide TRTA-P33 could specifically target MCF7 breast cancer cells with high expression of transferrin receptor 1.

The present disclosure is further illustrated below in conjunction with examples.

### Example 1: Adiposomes constructed using adiposome targeting peptide LTA-P33 can specifically target lung tissue

### 1. Preparation of adiposome targeting peptide LTA-P33

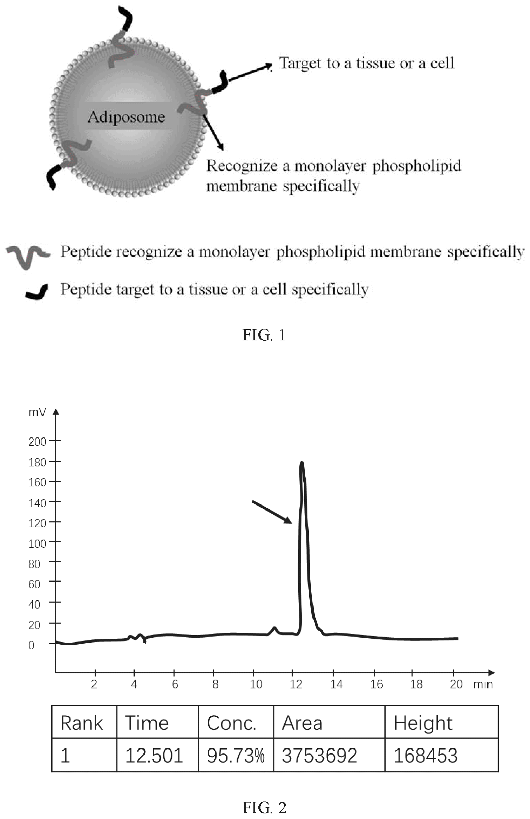

Adiposome targeting peptide LTA-P33 (lung-targeting adiposome peptide 33) was chemically synthesized by Shanghai GL Biochem Co., Ltd. The sequence of LTA-P33 was MELTIFILRLAIYILTFPLYLLNFLGLWCRGDK, wherein fragment having the amino acid sequence of MELTIFILRLAIYILTFPLYLLNFLGLW was AAMB, and the fragment having amino acid sequence of CRGDK was Nrp-B, which specifically recognized protein Neuropilin-1 (UniProt ID: P97333). The HPLC (high performance liquid chromatography) results of the chemically synthesized adiposome targeting peptide LTA-P33 are shown in FIG. 2, wherein the peak indicated by the arrow was the peak of LTA-P33 with a retention time of 12.5 min and the purity was 95.7%.

### 2. Construction of adiposomes containing adiposome targeting peptide LTA-P33

Adiposomes containing adiposome targeting peptide LTA-P33 were constructed according to the following method, and then silver staining and confocal laser scanning microscopy were used to verify the constructed adiposomes containing adiposome targeting peptide LTA-P33.
(1) LTA-P33 was dissolved in a mixed solution of methanol and chloroform (1:1, v/v) at a final concentration of 0.5 mg/ml.
(2) 20 µl of the solution obtained in (1) and 80 µl of 1,2-Dioleoyl-sn-glycero-3-phosphocholine solution (containing 2 mg DOPC) were mixed well, and a total volume of 100 µl was added to a microcentrifuge tube, and the solvent was dried by blowing with high-purity nitrogen gas.
(3) After completing step (2), 100 µl of phosphate-buffered saline (PBS) and 5 mg of triacylglycerol (TAG) or other neutral lipids were added to the microcentrifuge tube, followed by vortexing for 5 min (each cycle vortex for 10 s, stop for 5 s) to obtain a milky white lipid mixture 1 (i.e., initial prepared component). The lipid mixture 1 was centrifuged at 1000 g for 5 min, and the upper white band and the precipitate at the bottom of the tube were removed.
(4) After completing step (3), the lipid mixture 1 was centrifuged at 20000 g for 5 min (in practical applications, centrifugation at 18000-22000 g for 3-7 min is acceptable). After centrifugation, the precipitate at the bottom of the tube was removed, and the remaining emulsion in the tube was resuspended to obtain a milky white lipid mixture 2.
(5) After completing step (4), step 3 was repeated again to obtain the final adiposomes containing adiposome targeting peptide LTA-P33 (referred to as LTA adiposomes, i.e., lung-targeting adiposomes, FIG. 3).

FIG. 3A shows the result of: SDS-PAGE protein gel electrophoresis analysis of adiposomes containing adiposome targeting peptide LTA-P33, wherein the arrow in the LTA adiposome lane indicates the adiposome targeting peptide LTA-P33 adsorbed to the adiposomes. FIG. 3B shows the results of confocal laser scanning microscopy of adiposomes containing adiposome targeting peptide LTA-P33, wherein panel a shows a bright ring-like structure which represents the adiposome targeting peptide LTA-P33. Panel b shows adiposomes stained red by the neutral lipid-specific dye LipidTox Red (shown as light gray in the figure), and the bright ring-like structure on the surface was the adiposome targeting peptide LTA-P33. The scale bar was 5 µm. Silver staining and confocal laser scanning microscopy results demonstrated that adiposomes containing adiposome targeting peptide LTA-P33, i.e., lung-targeting adiposomes, were successfully constructed.

### 3. Verification of lung targeting of the adiposomes containing adiposome targeting peptide LTA-P33 (referred to as LTA adiposomes, i.e., lung-targeting adiposomes)

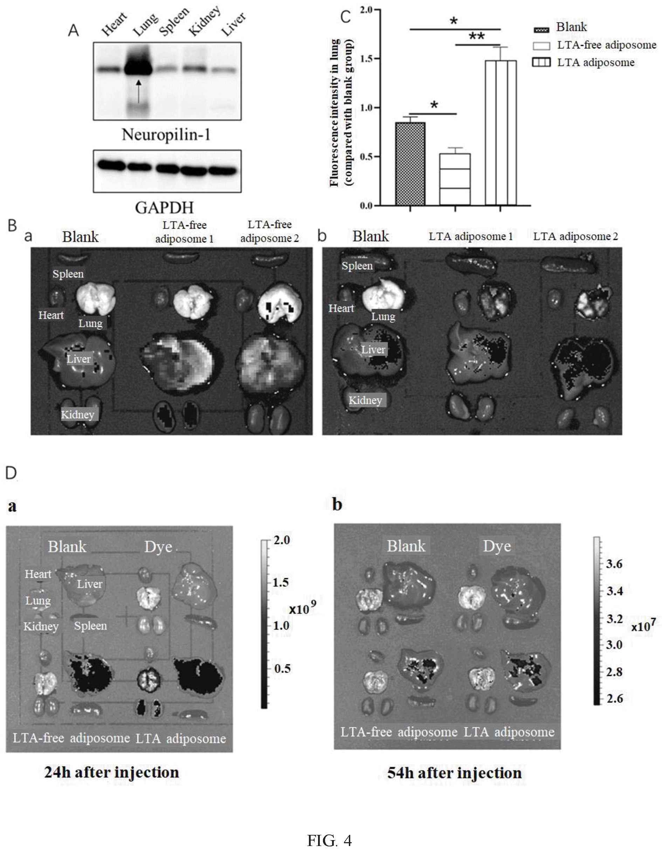

The adiposome targeting peptide LTA-P33 was specifically adsorbed to the adiposome surface via fragment A, and recognized tissue-specific protein Neuropilin-1 via fragment B, thereby achieving specific targeting of adiposomes. The amino acid sequence of fragment B was CRGDK, which could specifically recognize protein Neuropilin-1 (UniProt ID: P97333). Protein Neuropilin-1 was specifically highly expressed in lung tissue, while being expressed in very small amounts in other tissues such as heart and liver (FIG. 4 A).Adiposomes without adiposome targeting peptide LTA-P33 (LTA-free adiposomes) and adiposomes with adiposome targeting peptide LTA-P33 (LTA adiposomes) were injected into mice via tail vein, respectively. Three hours after injection, the mice were dissected, and various tissues were taken to identify the tissue distribution of LTA-free adiposomes and LTA-containing adiposomes. LTA-free adiposomes were almost entirely enriched in the liver (panel a of FIG. 4B), while LTA adiposomes were almost entirely enriched in lung tissue (panel b of FIG. 4B). Lung fluorescence statistics results showed that LTA adiposomes exhibit specific targeting capability and could target lung tissue (FIG. 4 C). In addition, 24 hours after injection, LTA adiposomes still remained in lung tissue ( FIG. 4 D).

### Example 2: Adiposomes constructed using adiposome targeting peptide TRTA-P33 specifically target MCF7 breast cancer cells with high expression of transferrin receptor

### 1. Preparation of adiposome targeting peptide LTA-P33

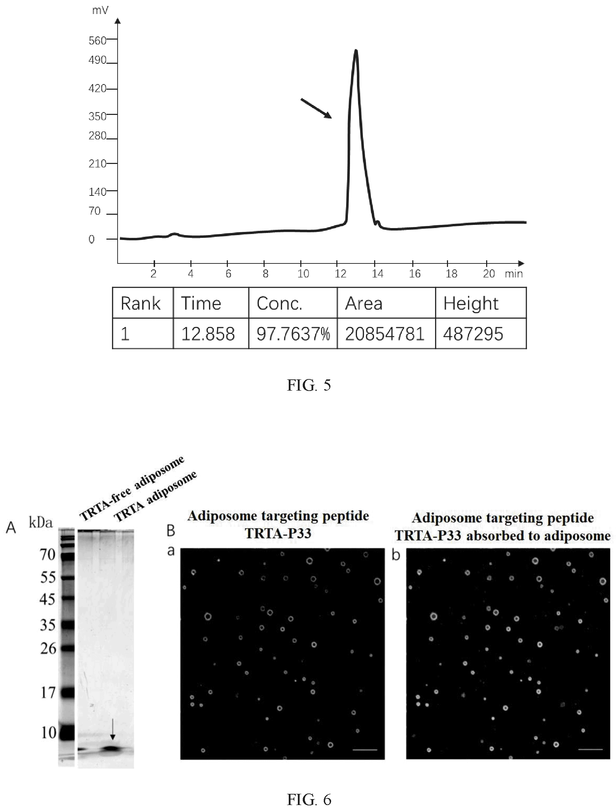

The adiposome targeting peptide TRTA-P33 (Transferrin receptor 1-targeting adiposome peptide 33) was chemically synthesized by Shanghai GL Biochem Co., Ltd . The sequence of TRTA-P33 was MGIFSRFADIVNANINALLEKTHRPPMWSPVWP, and adiposomes constructed using the peptide TRTA-P33 could specifically target MCF7 breast cancer cells with high expression of transferrin receptor. The fragment having amino acid sequence of MGIFSRFADIVNANINALLEK was PspA-H1, and the fragment having amino acid sequence of THRPPMWSPVWP was Trf-B, which specifically recognized the peptide of protein Transferrin receptor 1 (UniProt ID: P02786). The HPLC (high performance liquid chromatography) results of the chemically synthesized adiposome targeting peptide TRTA-P33 were shown in FIG. 5, wherein the peak indicated by the arrow was the peak of TRTA-P33 with a retention time of 12.8 min and a purity of 97.6%.

### 2. Construction of adiposomes containing adiposome targeting peptide TRTA-P33 (abbreviated as TRTA adiposomes, i.e., transferrin receptor-targeting adiposomes).

The preparation method was the same as described in Example 1, and the final adiposomes containing adiposome targeting peptide TRTA-P33 were obtained (FIG. 6). FIG. 6A shows the SDS-PAGE protein gel electrophoresis analysis of adiposomes containing adiposome targeting peptide TRTA-P33, where the arrow in the TRTA adiposome lane indicates the adiposome targeting peptide TRTA-P33 adsorbed to the adiposomes. FIG. 6B shows the confocal laser scanning microscopy results of adiposomes containing adiposome targeting peptide TRTA-P33, where the bright ring-like structure shown in panel a is the adiposome targeting peptide TRTA-P33. Panel b shows the adiposomes stained red by the neutral lipid-specific dye LipidTox Red (shown as light gray in the figure), with the bright ring-like structure on the surface being the adiposome targeting peptide TRTA-P33. The scale bar is 5 µm. The silver staining and confocal laser scanning microscopy results demonstrated that adiposomes containing adiposome targeting peptide TRTA-P33, i.e., transferrin receptor-targeting adiposomes, were successfully constructed.

### 3. Verification of targeting of adiposomes containing adiposome targeting peptide TRTA-P33 to MCF7 breast cancer cells with high expression of transferrin receptor (abbreviated as TRTA adiposomes, i.e., transferrin receptor-targeting adiposomes)

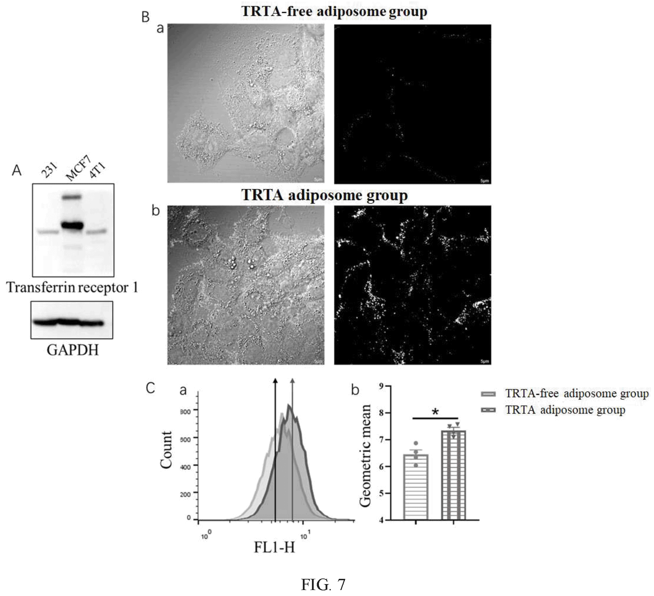

The adiposome targeting peptide TRTA-P33 was specifically adsorbed to the adiposome surface via the fragment A and specifically recognized the specific protein Transferrin receptor 1 via fragment B, thereby achieving specific targeting of adiposomes. The amino acid sequence of fragment B was THRPPMWSPVWP, which could specifically recognize the protein Transferrin receptor 1 (UniProt ID: P02786). The protein Transferrin receptor 1 was highly expressed in MCF7 breast cancer cells, while expressed at very low levels in 231 and 4T1 cancer cells (FIG. 7 A). The adiposomes without adiposome targeting peptide TRTA-P33 (non-TRTA adiposomes) and adiposomes with TRTA-P33 (TRTA adiposomes) were incubated with MCF7 cells, respectively. After two hours, the culture medium was removed, the cells were washed three times with PBS, and then confocal laser scanning microscopy observation and fluorescence signal statistics by flow cytometry were performed. Non-TRTA adiposomes were hardly observed in MCF7 cells (panel a of FIG. 7 B), whereas TRTA adiposomes were significantly accumulated in MCF7 cells (panel b of FIG. 7B). Flow cytometry results showed that the fluorescence peak of MCF7 cells treated with TRTA adiposomes was significantly shifted to the right (panel a of FIG. 7C). Statistical results showed that in MCF7 cells, the signal of TRTA adiposomes was significantly higher than that of non-TRTA adiposomes (panel b of FIG. 7C). These results demonstrated that adiposomes containing adiposome targeting peptide TRTA-P33 specifically targeted MCF7 breast cancer cells with high expression of transferrin receptor.

The foregoing are merely preferred embodiments of the present disclosure. It should be noted that, for those of ordinary skilled in the art, without departing from the principles of the present disclosure, several improvements and modifications can be made, and these improvements and modifications should also be regarded as the scope of protection of the present disclosure.

## Claims

1. A fusion protein comprising fragment A and fragment B;
wherein fragment A recognizes and targets a monolayer phospholipid membrane; and
fragment B recognizes and targets a tissue or a cell.

2. The fusion protein according to claim 1, wherein the structure of the fusion protein is:
[(fragment A)x-(fragment B)y]z or [(fragment B)x-(fragment A)y]z;
wherein x, y, and z are each independently an integer other than 0.

3. The fusion protein according to claim 1 or 2, wherein the fusion protein further comprises a linker.

4. The fusion protein according to claim 3, wherein the linker is selected from the group consisting of (GGGGS)n, Gn, (EAAAK)n, (XP)n, A(EAAAK)₄ALEA(EAAAK)₄A, PAPAP, VSQTSKLTRAETVFPDV, PLGLWA, TRHRQPRGWE, AGNRVRRSVG, RVLAEA, EDVVCCSMSY, EDVVCCSMSY, and GGIEGRGS, wherein n is an integer other than zero.

5. The fusion protein according to any one of claims 1 to 4, wherein the monolayer phospholipid membrane comprises one or more selected from the group consisting of phospholipids, functional polar lipids, and cationic lipids;
the phospholipid is one or more selected from the group consisting of phosphatidylcholine, phosphatidylethanolamine, phosphatidylinositol, phosphatidylserine, phosphatidylglycerol, phosphatidic acid, cardiolipin, and sphingomyelin;
the functional polar lipid is one or more selected from the group consisting of PEGylated sterol, biotinylated sterol, amino acid-modified sterol, peptide-modified sterol, polysaccharide-modified sterol, nucleic acid-modified sterol, PEGylated phospholipid, biotinylated phospholipid, amino acid-modified phospholipid, peptide-modified phospholipid, polysaccharide-modified phospholipid, and nucleic acid-modified phospholipid; and
the cationic lipid is one or more selected from the group consisting of (2,3-dioleoyl-propyl)-trimethylammonium-chloride, (2,3-dioleoyl-propyl)- trimethylammonium, 2,3-dioleoyloxy-N-[2-(sperminecarbamoylamino)ethyl]-N,N-dimethyl-1-propanamine hydrochloride, 1,2-dioleoyl-sn-glycero-3-[(N-(5-amino-1-carboxypentyl)iminodiacetic acid)succinyl](nickel salt), and 3β-[N-(N',N'-dimethylaminoethane)-carbamoyl]cholesterol hydrochloride.

6. The fusion protein according to any one of claims 1 to 4, wherein the fragment A is at least one selected from the group consisting of AAMB, ALDI, CYB5R3-N, LDAMP1, HSD17B13-N28, MDT-28-P, MLDS-P, DHS-3-P, HSD17B11-N28, PspA-H1, Vipp1-H1, Snf7-H1, Chmp1B-H1, PB, PE, and PF.

7. The fusion protein according to any one of claims 1 to 4, wherein the tissue or cell is from a tumor, a pathogen-infected organ, a lesion of a metabolic disease, and/or a healthy tissue or cell of a human or an animal body.

8. The fusion protein according to claim 7, wherein the fragment B is at least one selected from the group consisting of Nrp-B, Trf-B, LDLR-B, ErbB2-B, CXCR4-B, GRP78-B, and Soma-B.

9. The fusion protein according to any one of claims 1 to 4, wherein the fusion protein is AAMB-Nrp-B or PspA-H1-Trf-B.

10. A biological material comprising any one of:
I) a nucleic acid encoding the fusion protein according to any one of claims 1 to 9;
II) an expression unit comprising the nucleic acid according to I);
III) a vector comprising the nucleic acid according to I) or the expression unit according to II);
IV) a host cell transformed or transfected with the vector according to III); and
V) an expression product of the host cell according to IV).

11. A method for producing the fusion protein according to any one of claims 1 to 9, comprising
culturing the host cell according to claim 10 to obtain the fusion protein;
connecting the fragment A and the fragment B via a biological and/or chemical method; or
synthesizing the fusion protein according to any one of claims 1 to 9 by chemical synthesis.

12. Use of the fusion protein according to any one of claims 1 to 9 in the manufacture of a targeting adiposome.

13. A targeting adiposome, which is produced by raw materials comprising the fusion protein according to any one of claims 1 to 9, a polar lipid, and a neutral lipid.

14. The targeting adiposome according to claim 13, wherein
the polar lipid comprises one or more selected from the group consisting of phospholipids, functional polar lipids, and cationic lipids;
the phospholipid is one or more selected from the group consisting of phosphatidylcholine, phosphatidylethanolamine, phosphatidylinositol, phosphatidylserine, phosphatidylglycerol, phosphatidic acid, cardiolipin, and sphingomyelin; and
the functional polar lipid is one or more selected from the group consisting of PEGylated sterol, biotinylated sterol, amino acid-modified sterol, peptide-modified sterol, polysaccharide-modified sterol, nucleic acid-modified sterol, PEGylated phospholipid, biotinylated phospholipid, amino acid-modified phospholipid, peptide-modified phospholipid, polysaccharide-modified phospholipid, and nucleic acid-modified phospholipid;
the cationic lipid is one or more selected from the group consisting of (2,3-dioleoyl-propyl)-trimethylammonium-chloride, (2,3-dioleoyl-propyl)- trimethylammonium, 2,3-dioleoyloxy-N-[2-(sperminecarbamoylamino)ethyl]-N,N-dimethyl-1-propanamine hydrochloride, 1,2-dioleoyl-sn-glycero-3-[(N-(5-amino-1-carboxypentyl)iminodiacetic acid)succinyl](nickel salt), and 3β-[N-(N',N'-dimethylaminoethane)-carbamoyl]cholesterol hydrochloride; and
the neutral lipid is one or more selected from the group consisting of triglycerides, wax esters, steryl esters, sterol esters, retinol esters, and fat-soluble vitamins.

15. The targeting adiposome according to claim 13 or 14, wherein the raw materials further comprise a solvent, wherein the solvent comprises an organic solvent and a buffer.

16. The targeting adiposome according to claim 13 or 14, wherein the raw materials further comprise a drug.

17. A method for producing the targeting adiposome according to any one of claims 13 to 16, comprising
dissolving the fusion protein according to any one of claims 1 to 9 to obtain solution A;
dissolving a polar lipid to obtain solution B;
mixing the solution A and the solution B, drying, redissolving, mixing with a neutral lipid, and performing centrifugal separation to obtain a product containing the targeting adiposome.

18. The method according to claim 17, wherein a molar ratio of the polar lipid to the fusion protein is (100 to 100,000):(1 to 10).

19. Use of the targeting adiposome according to any one of claims 13 to 16 or the targeting adiposome obtained by the method according to claim 17 or 18 in the manufacture of a medicament or vaccine for preventing and/or treating a disease.

20. The use according to claim 19, wherein the disease is a cancer, wherein the cancer is selected from the group consisting of breast cancer, lung cancer, kidney cancer, laryngeal cancer, liver cancer, muscle tissue cancer, blood cancer, bone cancer, brain cancer, neck cancer, oral or nasal mucosal cancer, bladder cancer, central nervous system cancer, cervical cancer, colon cancer, endometrial cancer, external genitalia cancer, esophageal cancer, gallbladder cancer, gastrointestinal cancer, genitourinary cancer, head cancer, ovarian cancer, pancreatic cancer, prostate cancer, skin cancer, spleen cancer, small intestine cancer, large intestine cancer, stomach cancer, testicular cancer, and thyroid cancer.

21. A drug or vaccine comprising a pharmaceutically acceptable excipient and the targeting adiposome according to any one of claims 13 to 16, or the targeting adiposome produced by the method according to claim 17 or 18.

22. The drug or vaccine according to claim 21, wherein a dosage form thereof is an oral preparation, an inhalant, or an injection;
the oral preparation is a tablet, a pill, an oral liquid, a capsule, a syrup, a dropping pill, or a granule;
the inhalant is an inhalation aerosol, an inhalation powder, and a liquid preparation for use in a nebulizer; and
the injection is an injection solution or a powder for injection.

23. A method for preventing and/or treating a disease, comprising administering the drug or vaccine according to claim 22 to the subject in need thereof.
